# EUROPEAN PATENT SPECIFICATION

(11) **EP 0 353 990 B1**
(45) Date of publication and mention of the grant of the patent: **30.11.1994**
(21) Application number: 89307803.0
(22) Date of filing: 01.08.1989
(51) Int. Cl.: C07C 31/27, C07C 29/56

(54) **Process for preparing trans-1, 4 cyclohexanedimethanol**
Herstellungsverfahren von Trans-1,4-cyclohexandimethanol
Procédé de préparation du trans-1,4-cyclohexanediméthanol

(30) Priority: 01.08.1988 JP 190726/88; 27.12.1988 JP 327811/88; 10.04.1989 JP 87991/89
(43) Date of publication of application: 07.02.1990
(73) Proprietor: TOWA CHEMICAL INDUSTRY CO., LTD., Chuo-ku, Tokyo 104 (JP)
(72) Inventor: Tateno, Yoshiaki, Omiya-shi Saitama, 417 (JP); Yoneda, Susumu, Fuji-shi Shizuoka, 417 (JP); Okamoto, Naoki, Fuji-shi Shizuoka, 417 (JP); Ishii, Yoshibumi, Fuji-chi Shizuoka, 417 (JP); Kato, Kazuaki, Kitakatsushika-gun Saitama, 342 (JP)
(74) Representative: Holmes, Michael John

(56) References cited:
- GB-A- 957 667
- US-A- 2 917 549
- Beilsteins Handbuch der Organischen Chemie, 4th edition, 3rd supplement, Volume 6, Part 6, p. 642, item "1,4-bis-hydroxymethyl-cyclohexan".

## Description

The present invention relates to a process for preparing trans-1,4-cyclohexanedimethanol and a powder thereof, which is useful as a starting material for polyester resin, polyester synthetic fibre, polyester coating materials and the like, or as a chemical intermediate.

1,4-cyclohexanedimethanol (hereinafter referred to as 1,4-CHDM) is industrially prepared by distillation, for example, by hydrogenating 1,4-cyclohexanedicarboxylic acid dimethylester in the presence of a copper-chromium catalyst and then distilling the hydrogenated compound.

1,4-CHDM thus prepared usually comprises about 70% by weight of trans-1,4-CHDM and about 30% by weight of cis-1, 4-CHDM, and it is waxy or massecuite-like at room temperature.

The melting point of high purity cis-1,4-CHDM is lower than room temperature, it is consequently a liquid at room temperature; on the contrary, the melting point of high purity trans-1,4-CHDM is 60 to 64°C, and it is a solid at room temperature.

Polyester resins made from trans-1,4-CHDM have high glass transition temperatures and high softening points, and their resin properties are superior. Therefore, trans-1,4-CHDM is preferable as the starting material for polyester products.

Conventional processes for preparing trans-1,4-CHDM are described below:
(a) a mixture of cis- and trans-1,4-CHDM is converted to the trimethylsilyl derivatives and the compounds are separated by diglycerol column chromatography, as disclosed in Acta Pharmaceutica Suetica 5(5), pp 449-456 (1968).
(b) A mixture of cis- and trans-1,4-CHDM is separated by cyclodextrin-combined silica gel column chromatography, as disclosed in Journal of Liquid Chromatography 10(6) pp 1077-1084 (1987).
(c) A mixture of cis- and trans-1,4-CHDM is converted to the dibenzoate derivatives, and the compounds are selectively crystallized, utilizing the difference of crystallization, and then hydrolyzed to obtain trans-1,4-CHDM, as disclosed in Journal of the Chemical Society pp 404-407 (1953).

However, according to the conventional processes described above, high purity trans-1,4-CHDM may be obtained in only a small amount by distillation, wherein the majority of distillate is a mixture of trans- and cis-1,4-CHDM, containing about 30% of cis-1,4-CHDM, the mixture having a melting point of about 40°C.

1,4-CHDM containing about 30% of cis-1,4-CHDM has the following disadvantages:
In the case of commercial transport, expensive drum cans are needed, because the compound is waxy or massecuite-like; it requires previous heating at temperatures of 50-60°C prior to use, and it is decomposed or coloured by heating or melting, therefore its use is restricted; heating or melting is expensive and therefore, the product made from it becomes costly.

Further, conventional processes of preparing trans-1,4-CHDM as shown in the above methods (a)-(c) are analytical or laboratory processes, and suffer the problems of low yield, high cost and complicated manufacturing processes. Therefore, the conventional processes are not suitable for commercial production of trans-1,4-CHDM or powders thereof.

In view of the above-mentioned background, there is a need to obtain commercially useful trans-1,4-CHDM and powdered trans-1,4-CHDM.

The inventors found that high purity trans-1,4-CHDM may be obtained in good yield by distilling a mixture of cis- and trans-1,4-cyclohexanedimethanol in the presence of a base and that powdered trans-1,4-CHDM can be obtained by pulverizing the distillate.

The present invention thus provides a process for obtaining trans-1,4-cyclohexanedimethanol wherein a mixture of cis- and trans-cyclohexanedimethanol is heated in the presence of a base and subjected to distillation during said heating.

In order to obtain powdered trans-1,4-cyclohexanedimethanol, the process of the invention may be effected to produce a distillate containing predominantly trans-1,4-cyclohexanedimethanol and the distillate then pulverised (after solidification).

The mixture of cis- and trans-1,4-CHDM used in this invention may be obtained by conventional methods.

In the present invention, a solvent such as water, methanol or ethanol may be used, and a solution of 1,4-CHDM having a concentration of 50 to 100% by weight is preferably used. However, the reaction is more preferably carried out in the absence of solvent.

The bases used in the process of the present invention include alkali compounds, for example hydroxides of alkali metals such as sodium, potassium and lithium, hydroxides of alkaline earth metals and alcoholates such as sodium methoxide and sodium ethoxide, and amines such as ethanolamine.

The base is preferably used in an amount of 0.1 to 5% by weight relative to the amount of 1,4-CHDM. When the base is used in an amount of less than 0.1%, trans-1,4-CHDM may be obtained is relatively small yield, and when the base is used is an amount of more than 5%, ether-linked polymers tend to be obtained.

The base may be added in powder form. However, bases are preferably added in solution in a small amount of water or organic solvents, or in an alcohol solution, because alkali compounds are only slightly soluble in 1,4-CHDM.

The base may conveniently be added before or during distillation.

After the distillate containing trans-1,4-CHDM in a high percentage is obtained by adding the base during distillation, further base may be added and the mixture heated to obtain trans-1,4-CHDM by distillation.

Alternatively, the base may be added before distillation, and the mixture heated under the following conditions:

### Heating temperature:

150 to 200°C, preferably 160 to 200°C.

When the reaction is carried out below 150°C, a relatively long time is required to obtain a large amount of trans-1,4-CHDM.

When the reaction is carried out above 250°C, ether-linked by-products tend to be produced in a large amount.

### Reaction time:

The reaction time depends on the heating temperature and the type and quantity of base. For example, when the reaction is carried out for one hour at a temperature of 160°C and in the presence of 2% by weight of base, or when the reaction is carried out for 30 minutes at a temperature of 200°C and in the presence of 0.5% by weight of base, trans-1,4-CHDM may be obtained in the greatest quantity.

The distillation is preferably carried out at temperatures of 150 to 200°C, and under a reduced pressure of 133 to 6650 Pa (1 to 50 mmHg).

The distillation may be carried out under a reduced presure of lower than 133 Pa (1 mmHg) or higher than 6650 Pa (50 mmHg). However, when the pressure is lower than 133 Pa (1 mmHg), expensive equipment is required, and when the pressure is higher than 6650 Pa (50 mmHg), higher temperatures are required for distillation, and decomposition or etherification tends to occur.

For example, when 1,4-cyclohexanedicarboxylic acid dimethylester is reduced in the presence of a copper-chromium catalyst to obtain reaction products containing principally 1,4-CHDM, the products usually contain about 70% of trans-1,4-CHDM. When said reduction is carried out in the presence of 0.5% by weight (per reaction mixture) of NaOH at a temperature of 180°C for about 60 minutes, the products containing about 85% of trans-1,4-CHDM can be obtained, and further, the products containing about 90% of trans-1,4-CHDM can be obtained by distillation.

On the other hand, when alkali is added during the distillation, the product containing about 95% of trans-1,4-CHDM can be obtained in a yield of 95%.

The distillation may conveniently be carried out by batch or continuous distillation, and preferably by continuous distillation with a rectification tower having "number of theoretical plates" of ten to twenty plates.

1,4-CHDM containing about 85% of trans-1,4-CHDM has a melting point of about 60°C, and is a hard solid at low temperatures. Therefore, powdered 1,4-CHDM can be obtained by kneading and shaping 1,4-CHDM containing predominantly trans-1,4-CHDM under cooling, or by cooling to a solid state and then cutting it.

Polyester resins and derivatives thereof having superior qualities such as higher glass transition point may conveniently be prepared by using 1,4-CHDM or powdered 1,4-CHDM containing predominantly trans-1,4-CHDM.

Polyester copolymers of higher heat-stability may be obtained by reacting a glycol containing 50 to 97 mol% of 1,4-CHDM which comprises 80% or more of trans-1,4-CHDM, preferably 90% or more of trans-1,4-CHDM, and a glycol containing 3 to 50 mol% of ethylene glycol, with a dicarboxylic acid such as terephthalic acid, as compared to polyester copolymers obtained from 1,4-CHDM containing less than 80% of trans-1,4-CHDM.

In the preparation of the above-mentioned polyester copolymers, a fibre reinforcing agent, flame retarder, colouration-preventing agent, antistatic agent, heat-resisting agent, weather proofing agent and the like may be added, if necessary, to improve the qualities of polyester resins and the derivatives thereof.

Consequently, derivatives such as polyester resins having superior qualities in heat resistance, fire resistance, weather-proofing, deformation resistance, antistatic properties and the like can conveniently be prepared by using trans-1,4-CHDM or powders thereof obtained by the process of the present invention or by adding thereto various agents as shown in the above.

As mentioned above, trans-1,4-CHDM can be prepared at lower expense and higher yield by the process of the present invention.

By the process of the present invention, powdered trans-1,4-CHDM can be made advantageously from a commercial point of view. It is a solid at room temperature, easy to handle and capable of storage in cheap containers.

Further, by using trans-1,4-CHDM or powders thereof obtained by the present invention, or by using the products with added improving agents, derivatives such as polyester resins having superior qualities in heat resistance, fire resistance, weather resistance, deformation resistance, antistatic property and the like can be manufactured, and consequently the range of use of 1,4-CHDM can be increased.

The present invention will be illustrated by the following non-limiting Examples.

### Preparation of Raw materials (1,4-CHDM)

250 kg of 1,4-cyclohexanedicarboxylic acid dimethylester and 25 kg of copper-chromium catalyst (C-5, manufactured by Sakai Chemical Co., Ltd.) were agitated in an autoclave (volume: 550 liter) at a temperature of 260°C, and after hydrogen had been absorbed, the catalyst was filtered off. The filtrate obtained was distilled to yield Raw material (a) and Raw material (b).

Raw material (a) and Raw material (b) were analyzed by gas chromatography. Raw material (a) comprised 45% of cis-1,4-CHDM and 55% of trans-1,4-CHDM, and Raw material (b) comprised 26% of cis-1,4-CHDM and 74% of trans-1.4-CHDM.

Raw material (a) was rice cake like, and Raw material (b) was a waxy solid. Both materials (a) and (b) could not be crushed.

### EXAMPLES

### Example 1

3 kg of Raw material (b) were placed in a 5 litre flask and were distilled in a rectification tower (having "number of theoretical plates" of 20 plates, length of one meter and diameter of 30 mm, and containing Raschig rings) under the conditions of a reduced pressure of 1330 Pa (10 mmHg) and temperatures of 152 to 163°C. 0.72 kg of Distillate Ea and 2.00 kg of Distillate Eb were separately obtained.

Distillate Ea comprised 3.4% cis-1,4-CHDM and 96.6% trans-1,4-CHDM, while Distillate Eb comprised 33.2% of cis-1,4-CHDM and 66.8% trans-1,4-CHDM.

A mixture of 2 kg of Distillate Eb and 80 g of a 50% aqueous solution of NaOH was placed in a 3 litre flask with an agitator, slowly heated to 180°C then heated at a temperature of 180°C for 30 minutes, and then cooled to room temperature. The reaction mixture was neutralized by hydrochloric acid to obtain Substance F.

Substance F comprised 15.8% of cis-1,4-CHDM and 84.2% of trans-1,4-CHDM.

Substance F was placed in a 5 litre flask and was distilled in the same manner as that mentioned above to yield 0.64 kg of Distillate Ga and 1.10 kg of Distillate Gb.

Distillate Ga comprised 2.2% cis-1,4-CHDM and 97.8% trans-1,4-CHDM.

Distillate Gb comprised 22.2% cis-1,4-CHDM and 77.8% trans-1,4-CHDM.

### Example 2

A mixture of 3 kg of Raw material (b) and 60 g of a 50% aqueous solution of NaOH was placed in a 5 litre flask and was distilled in a rectification tower (having "number of theoretical plates" of 20 plates, length of one meter and diameter of 30 mm, and containing Raschig rings) under the conditions of a reduced pressure of 1330 Pa (10 mmHg) and temperatures of 153 to 162°C. 0.6 kg of Distillate Ha, 0.8 kg of Distillate Hb and 1.2 kg of Distillate Hc were obtained.

Distillate Ha comprised 3.6% cis-1,4-CHDM and 96.4% trans-1,4-CHDM. Distillate Hb comprised 2.4% cis-1,4-CHDM and 97.6% trans-1,4-CHDM. Distillate Hc comprised 2.2% cis-1,4-CHDM and 97.8% trans-1,4-CHDM.

### Example 3

A mixture of 800 g of Raw material (b) and 16 g of sodium methylate was distilled in a rectification tower (having "number of theoretical plates" of 20 plates, length of one meter and diameter of 30 mm, and containing Raschig rings) under the conditions of a reduced pressure of 5320 Pa (40 mmHg) and temperatures of 185 to 188°C. 500g of Distillate J were obtained.

Distillate J comprised 3.6% cis-1,4-CHDM and 96.4% trans-1,4-CHDM (by chromatographic analysis).

Distillate J was poured into a stainless steel tray at a temperature of 70°C, and then allowed to stand overnight. The solid thus obtained was cut with a frozen cutter (Type FZ, made by Shonan Industrial Co., Ltd.) to yield a white powder of trans-1,4-CHDM having a melting point of 62°C.

### Example 4

A mixture of 800 g of Raw material (a) and 8 g of NaOH was placed in a 1 litre stainless steel autoclave and heated at a temperature of 180°C for two hours. After cooling, the reaction mixture was distilled in the same manner as that described in Example 3 to yield Distillate K.

Distillate K was poured into a tray, cooled to a solid and then crushed to obtain a white powder. The powder comprised 3.2% cis-1,4-CHDM and 96.8% trans-1,4-CHDM and had a melting point of 62°C.

### Example 5

A mixture of 800 g of Raw material (b) and 6 g of sodium alcoholate was distilled in a rectification tower (having "number of theoretical plates" of 10 plates, length of 0.5 m, and diameter of 30 mm, and containing Raschig rings) under the conditions of a reduced pressure of 5320 Pa (40 mmHg) and temperatures of 185 to 188°C to yield 500 g of Distillate L.

After Distillate L was kneaded by a kneader having a jacket (Type PNV-1; made by Irie Shokai Co., Ltd.) for 10 minutes, and then allowed to stand overnight, the solid thus obtained was crushed with a frozen cutter to obtain a white powder of trans-1,4-CHDM. The powder comprised 9.7% cis-1,4-CHDM and 90.3% trans-1,4-CHDM, and had a melting point of 58°C.

### Example 6

A mixture of 800 g of Raw material (b) and 5 g of sodium alcoholate was distilled in a rectification tower (having "number of theoretical plates" of 10 plates, length of 0.5 m, and diameter of 30 mm, and containing Raschig rings) under the conditions of a reduced pressure of 5320 Pa (40 mmHg) and temperatures of 185 to 188°C to yield 500 g of Distillate M.

After Distillate M was kneaded by a kneader having a jacket for 10 minutes and allowed to stand for one hour, the solid thus obtained was shaped with a pelletizing device (Type EXD-100 made by Fuji Powdal Co., Ltd.) to yield white pellets having length of 10 to 50 mm and diameter of 3 mm. The pellets comprised 3.2% cis-1,4-CHDM and 96.8% trans-1,4-CHDM and had a melting point of 62°C.

## Claims

1. A process for obtaining trans-1,4-cyclohexanedimethanol from a mixture of cis- and trans-1,4-cyclohexanedimethanol characterised in that to obtain a product comprising at least 80% of trans-1,4-cyclohexanedimethanol a mixture of cis- and trans-1,4-cyclohexanedimethanol is heated at a temperature of about 150 to 200°C in the presence of a base; subjected to distillation under a reduced pressure of 133 to 6650 Pa (1 to 50mm Hg) during said heating; and the product of said distillation is cooled to form a solid.

2. A process as claimed in claim 1, wherein said mixture is heated at a temperature in the range 160 to 200°C.

3. A process as claimed in claim 1 or claim 2, wherein the base is an alkali metal hydroxide, an alkaline earth metal hydroxide, an alcoholate or an amine.

4. A process as claimed in any of the preceding claims, wherein the base is added in solution in water, in an organic solvent or in an alcohol.

5. A process as claimed in any of the preceding claims, wherein the base is added before or during distillation.

## Patentansprüche

1. Verfahren, um trans-1,4-Cyclohexandimethanol aus einem Gemisch von cis- und trans-1,4-Cyclohexandimethanol zu erhalten, dadurch gekennzeichnet, daß man, um ein Produkt zu erhalten, das mindestens 80 % trans-1,4-Cyclohexandimethanol umfaßt, ein Gemisch aus cis- und trans-1,4-Cyclohexandimethanol auf eine Temperatur von etwa 150 bis 200°C in Gegenwart einer Base erwärmt; das Gemisch einer Destillation unter einem verringerten Druck von 133 bis 6650 Pa (1 bis 50 mmHg) während des Erwärmens unterwirft; und das Produkt der Destillation unter Bildung eines Feststoffs abkühlt.

2. Verfahren nach Anspruch 1, wobei das Gemisch auf eine Temperatur im Bereich von 160 bis 200°C erwärmt wird.

3. Verfahren nach Anspruch 1 oder 2, wobei es sich bei der Base um ein Alkalimetallhydroxid, ein Erdalkalimetallhydroxid, ein Alkoholat oder ein Amin handelt.

4. Verfahren nach einem der vorstehenden Ansprüche, wobei die Base in einer Lösung in Wasser, einem organischen Lösungsmittel oder einem Alkohol zugegeben wird.

5. Verfahren nach einem der vorstehenden Ansprüche, wobei die Base vor oder während der Destillation zugegeben wird.

## Revendications

1. Un procédé pour obtenir du trans-1,4-cyclohexanediméthanol à partir d'un mélange de cis- et de trans-1,4-cyclohexanediméthanol, caractérisé en ce que, pour obtenir un produit comprenant au moins 80 % de trans-1,4-cyclohexanediméthanol, on chauffe un mélange de cis- et de trans-1,4-cyclohexanediméthanol à une température d'environ 150 à 200°C en présence d'une base ; on le soumet à une distillation sous une pression réduite comprise entre 133 et 6650 Pa (1 à 50 mm Hg) pendant ce chauffage ; et on refroidit le produit de cette distillation pour former un solide.

2. Un procédé tel que revendiqué dans la revendication 1, dans lequel ledit mélange est chauffé à une température comprise dans la plage allant de 160 à 200 °C.

3. Un procédé tel que revendiqué dans la revendication 1 ou la revendication 2, dans lequel la base est un hydroxyde de métal alcalin, un hydroxyde de métal alcalino-terreux, un alcoolate ou une amine.

4. Un procédé tel que revendiqué dans l'une des revendications précédentes, dans lequel la base est ajoutée en solution dans l'eau, dans un solvant organique ou dans un alcool.

5. Un procédé tel que revendiqué dans l'une des revendications précédentes, dans lequel la base est ajoutée avant ou pendant la distillation.
